# EUROPÄISCHE PATENTANMELDUNG

(11) **EP 2 407 185 A1**
(43) Veröffentlichungstag der Anmeldung: **18.01.2012**
(21) Anmeldenummer: 10075302.9
(22) Anmeldetag: 15.07.2010
(51) Int. Cl.: A61M 1/10

(54) **Radial komprimierbarer und expandierbarer Rotor für eine Pumpe mit einem Schaufelblatt**

(71) Anmelder: ECP Entwicklungsgesellschaft mbH, 12247 Berlin (DE)
(72) Erfinder: Töllner, Thomas, 12047 Berlin (DE); Scheckel, Mario, 13187 Berlin (DE)
(74) Vertreter: Golkowsky, Stefan

(57) **Zusammenfassung**

Die Erfindung bezieht sich auf einen radial komprimierbaren und expandierbaren Rotor (13, 13a, 13b) für eine Pumpe mit wenigstens einem Schaufelblatt (19, 20, 22, 36, 36a, 36b, 36c, 36d, 36e, 36f, 36g, 37, 37a, 37b, 37c, 37d, 37e, 37f, 74, 88, 89, 90), wobei das Schaufelblatt einen Schaufelblattkörper aufweist, dessen Material elastisch verformbar ist, sowie wenigstens eine Versteifungsstrebe (25, 26, 27, 30, 31, 32, 52, 53, 57, 58, 62, 63, 67, 95), die in das Material des Schaufelblattkörpers wenigstens teilweise eingebettet ist.

Zur Stabilisierung des Schaufelblattes sind die Streben in Größe, Form und Anordnung geeignet gestaltet und in geeignete Hohlräume des Schaufelblattkörpers integriert. Zusätzlich können zugfeste Elemente vorgesehen sein.

## Beschreibung

Die Erfindung liegt auf dem Gebiet des Maschinenbaus und der Mechanik und befasst sich mit Rotoren für Pumpen.

Rotorpumpen sind vielseitig einsetzbar und bei entsprechender Ausgestaltung und Drehzahl des Rotors sehr leistungsfähig. Grundsätzlich können die Rotoren als axial fördernde oder zentrifugal fördernde Rotoren ausgebildet sein und weisen entsprechende Schaufelblätter auf.

Von besonderem Interesse sind derartige Pumpen dann, wenn sie im Verhältnis zu ihrer Förderleistung dadurch sehr klein ausgebildet werden können, dass sie zum Transport komprimiert und für den Betrieb expandiert werden können. Eine spezielle Anwendung derartiger Pumpen im medizinischen Bereich betrifft die invasiven Pumpen, die in radial komprimiertem Zustand in den Körper eines Patienten hinein befördert und dort expandiert werden können, um die gewünschte Förderleistung zu erzielen.

Solche Pumpen können beispielsweise als herzunterstützende Blutförderpumpen eingesetzt werden und durch ein Blutgefäß in einen Körperhohlraum befördert werden, um dort radial expandiert zu werden. Dazu ist typischerweise sowohl der Rotor als auch, soweit vorhanden, ein entsprechendes Pumpengehäuse radial komprimierbar und expandierbar.

Eine solche Pumpe ist beispielsweise aus dem US-Patent 7 393 181 B2 bekannt. Dort sind bei einem Rotor mehrere Schaufeln einstückig mit einer Nabe hergestellt und können im Transportzustand an diese aufgrund ihrer Materialelastizität angelegt werden, um den Rotor radial zu komprimieren. Im Betrieb richten sich die Schaufeln auf, so dass die Förderleistung entsprechend erhöht wird. Das Material des dortigen Rotors muss sorgfältig bezüglich seiner Elastizitäts- und Verformbarkeitseigenschaften ausgewählt werden, um einerseits leicht an die Nabe andrückbar zu sein und andererseits im Betrieb ausreichende Kräfte auf die zu fördernde Flüssigkeit ausüben zu können.

Eine sehr ähnliche Pumpe ist aus der US-Patentanmeldung US 2008/0114339 A1 bekannt, wo ein entsprechender Rotor zusätzlich mit einem Gehäuse zusammenwirkt, das ebenfalls komprimierbar und expandierbar ist. Im Betrieb des Rotors soll das Gehäuse durch die Kraftwirkung der sich aufrichtenden Rotorschaufeln expandiert werden.

Vor dem Hintergrund des Standes der Technik liegt der vorliegenden Erfindung die Aufgabe zugrunde, einen Rotor für eine Pumpe zu schaffen, der mit möglichst geringem Kraftaufwand radial komprimierbar ist, der andererseits im Betrieb ebenso leicht expandierbar ist und im expandierten Zustand die für die Schubwirkung erforderliche Stabilität aufweist.

Die Aufgabe wird gemäß der Erfindung mit den Merkmalen des Patentanspruchs 1 gelöst.

Es ist dazu gemäß der Erfindung ein radial komprimierbarer und expandierbarer Rotor für eine Pumpe mit wenigstens einem Schaufelblatt vorgesehen, wobei das Schaufelblatt einen Schaufelblattkörper aufweist, dessen Material elastisch verformbar ist, sowie wenigstens eine Versteifungsstrebe, die in das Material des Schaufelblattkörpers wenigstens teilweise eingebettet ist.

Der erfindungsgemäße Rotor zeigt vorzugsweise eine Stützstruktur (= Streben), die keine geschlossene Randstruktur bzw. Randkurve aufweist. Das heißt, dass in der vorliegenden Erfindung vorzugsweise einzelne Streben bzw. Lamellen vorgesehen sind, die weitgehend unabhängig voneinander verformbar sind und dadurch einerseits eine gute Komprimierung des Rotors ermöglichen, andererseits aber auch eine ausreichende Stabilisierung/Steifigkeit im Pumpbetrieb ermöglichen. Der Schaufelblattkörper hat geometrieerhaltende Eigenschaften gegenüber Verformungen im Pumpbetrieb, die lokal durch Versteifungsstreben versteift werden.

Dabei wird die Oberfläche des Schaufelblattkörpers von den Streben nicht durchbrochen, so dass keine Störung des Fluidstroms hervorgerufen wird. Vorzugsweise ist die Schaufelblattstärke (d. h. die Gesamt dicke des Schaufelblattes einschließlich eventueller Hohlräume) mindestens so dick/groß wie die größte Dicke der hierzu benachbarten bzw. hiermit verbundenen Streben. Dadurch ist die jeweilige Strebe allseits vom Kunststoffmaterial des Schaufelblatts bedeckt, und es ergibt sich (anders als beispielsweise bei folienbespannten Metallgerüsten) eine homogene, strömungsgünstige Schaufeloberfläche.

Durch diese Konstruktion können die verschiedenen mechanischen Anforderungen an den Rotor bzw. das Schaufelblatt auf verschiedene Materialien bzw. konstruktive Elemente verteilt werden. Die Streben können derart gebaut und positioniert sein, dass sie die Komprimierung des Schaufelblattes vom ersten in den zweiten Zustand nicht behindern, dass sie jedoch andererseits den Rotor im dritten, expandierten Zustand hinreichend stabilisieren. Die Streben übernehmen damit die Spannungen, während das Material des Schaufelblattkörpers die erforderliche Dehnbarkeit aufweist. Insoweit kann durch die Verwendung eines Kompositwerkstoffs auch die Gesamtmasse und das Gesamtvolumen des Rotors bzw. des Schaufelblattes minimiert werden. Dies führt zu einem geringen Durchmesser im zweiten, komprimierten Zustand.

Typischerweise bestehen die Streben aus einem steiferen Material als der Schaufelblattkörper, beispielsweise einem steifen Kunststoff oder einer Metalllegierung bzw. einem Metall.

Die Konstruktion ist derart gestaltet, dass der Rotor ohne äußere Krafteinwirkung einen ersten Zustand einnimmt, von dem er einerseits durch eine radiale Kompression in einen zweiten, komprimierbaren Zustand zu Zwecken des Transportes überführbar ist, während er von dem ersten Zustand ebenso im Betrieb durch Aufrichten des Schaufelblattes / der Schaufelblätter in einen dritten, expandierten Zustand überführbar ist.

Dadurch, dass der erste Zustand des Rotors gegenüber dem Betriebszustand schon teilweise komprimiert ist, sollen die zur endgültigen Kompression notwendigen Radialkräfte möglichst minimiert werden. Zudem nimmt das Schaufelblatt bzw. nehmen die Schaufelblätter im ersten Zustand eine Vorzugsposition ein, die unter der Wirkung des Fluidgegendrucks für ein Aufrichten der Schaufelblätter sowie dafür sorgt, dass im zweiten, komprimierten Zustand ihre Position optimiert ist.

Der Rotor kann vorteilhaft nabenlos ausgestaltet sein, und die Streben können sich bezüglich der Rotationsachse in Radialrichtung von einem ersten Achsabstand bis zu einem zweiten Achsabstand erstrecken. Dies bedeutet, dass die Streben sich radial nach innen nicht bis zur Rotationsachse erstrecken müssen, sondern in einem endlichen Abstand von dieser beginnend sich im Schaufelblatt radial nach außen erstrecken. Sie können radial außen bis zum Ende des Schaufelblattes verlaufen oder in einem zweiten Achsabstand vor dem Ende des Schaufelblattes enden. Das Schaufelblatt selbst kann geteilt sein und den rotationsachsnahen Bereich frei lassen, oder es kann sich als eine einzige durchgehende Schaufel über den zentralen Bereich hinaus, beispielsweise als ein einzelnes spiralförmiges Blatt, erstrecken.

Andererseits kann eine Nabe vorgesehen sein, mit der das Schaufelblatt verbunden und gegenüber der das Schaufelblatt schwenkbar ist. In diesem Fall ist das Einleiten der Antriebskräfte in das Schaufelblatt durch die Nabe vereinfacht möglich, während bei einem nabenlosen Rotor die Antriebskraft stirnseitig in die Schaufelblätter eingeleitet werden muss.

Vorteilhaft kann wenigstens eine Strebe, insbesondere auch mehrere oder alle Streben, sich bis radial in die Nabe hinein erstrecken. Die Streben können beispielsweise als einzelne Körper oder als ein verbundenes Gerüst in den Nabenkörper eingesteckt oder in eine Nut des Nabenkörpers eingezogen werden, um das Stützgerüst des Schaufelblattes zu bilden. Eine derartige nutenförmige Ausnehmung des Nabenkörpers verläuft typischerweise helixartig an seiner Mantelfläche entlang. Im Querschnitt kann die Nut beispielsweise schwalbenschwanzförmig ausgebildet sein, um die Streben zuverlässig zu halten.

Es kann vorteilhaft allerdings auch vorgesehen sein, dass wenigstens eine Strebe, insbesondere mehrere Streben oder alle Streben, sich radial von einem ersten Achsabstand außerhalb der Nabe bis zu einem zweiten Achsabstand erstrecken. In diesem Fall kann beispielsweise das Anklappen des Schaufelblattes an die Nabe erleichtert sein. Jedoch ist auch bei Streben, die sich in den Nabenkörper hinein erstrecken oder einstückig durch die Nabe hindurch verlaufen, ein Anklappen durch entsprechend optimierte Gestaltung gut möglich.

Die Streben können untereinander alle oder in Gruppen miteinander verbunden sein, wobei sie vorteilhaft innerhalb der rotationsachsnahen Hälfte ihrer radialen Ausdehnung, im expandierten Zustand des Rotors betrachtet, miteinander verbunden sein können. Insbesondere können sie auch an ihren rotationsachsnahen Enden miteinander verbunden sein, vorteilhaft auch ausschließlich dort. Es kann auch vorgesehen sein, dass die Streben jeweils paarweise an ihren rotationsachsfernen Enden miteinander verbunden sind und somit Schlaufen bilden, wobei die Schlaufen jeweils untereinander im achsnahen Bereich verbunden sind. Hierdurch ergibt sich eine gute Versteifung des Schaufelblattes in Richtung senkrecht zur Schubbelastung und eine gute Verformbarkeit innerhalb der Ebene des Schaufelblattes, so dass im Betrieb eine gute Stabilisierung durch die Streben ermöglicht wird, jedoch zur Kompression die Streben eine entsprechende Beweglichkeit im achsfernen Bereich des Schaufelblattes untereinander aufweisen können. Die Streben können als strangförmige Körper, beispielsweise in Form eines Drahtes, ausgebildet sein, jedoch auch als flache Teile eines blechartigen Körpers. In einer vorteilhaften Ausprägung können sie beispielsweise aus einem vollen Blech ausgestanzt oder durch entsprechende Schneidetechniken, beispielsweise Laserschneiden, Ätzen oder Erodieren, ausgeschnitten sein.

Vorteilhaft sind die Streben mit dem Material des Schaufelblattkörpers umspritzt, umgossen oder in flüssigem Zustand benetzt, wobei entsprechend eine nachfolgende Härtung vorgesehen sein kann.

Weiterhin kann vorteilhaft vorgesehen sein, dass die Streben in Ausnehmungen des Schaufelblattkörpers angeordnet sind, welche in Längs- und/oder in Querrichtung der jeweiligen Strebe größer sind als die Außenmaße der Strebe(n).

Dadurch, dass die Ausnehmungen im Schaufelblattkörper in wenigstens einer Ausdehnungsrichtung größer sind als die Maße der jeweiligen Strebe, kann die Strebe in den verschiedenen Zuständen des Rotors in unterschiedlichen Positionen in der jeweiligen Ausnehmung liegen. Damit lassen sich unterschiedliche Versteifungsgrade des Schaufelblattes in den verschiedenen Zuständen realisieren, so dass einerseits das Schaufelblatt in einer ersten Position im gestreckten Zustand des Schaufelblattes eine starke Versteifungswirkung ausüben kann, andererseits dieselbe Strebe sich beim Komprimieren des Rotors innerhalb ihrer Ausnehmung derart umlagern kann, dass sie die Kompression nur unwesentlich behindert.

Es kann vorteilhaft auch vorgesehen sein, dass wenigstens eine Strebe auf ihrer der Druckseite des Schaufelblattes zugewandten Fläche und/oder auf ihrer der Saugseite zugewandten Fläche an dem Material des Schaufelblattkörpers haftet. Beispielsweise kann, wenn die Strebe nur auf der der Saugseite zugewandten Fläche mit dem Material des Schaufelblattkörpers haftend verbunden ist und das Material des Schaufelblattkörpers inkompressibel ist, eine hohe Steifigkeit im gestreckten Zustand erreicht werden. Es kann je nach den Erfordernissen der Konstruktion die jeweilige Strebe nur auf der der Saugseite oder der Druckseite zugewandten Fläche haftend mit dem Schaufelblattkörper verbunden sein, oder die Strebe kann rundum an allen ihren Flächen am Schaufelblattkörper haften.

In einer weiteren Ausführungsform kann die Haftung zwischen Schaufelblattkörper und Strebe(n) unterschiedlich ausgebildet sein. Beispielweise kann der Rotor so gestaltet werden, dass der Schaufelblattkörper im Bereich der Nabe fest mit den Streben verbunden ist, wogegen er im Bereich der Streben fern der Nabe nicht haftet und somit beim Komprimieren gegenüber den Streben gleiten kann. Dies kann z. B. durch den lokalen Einsatz von Haft- bzw. Trennmitteln bzw. unterschiedliche Oberflächengestaltung (d. h. Rauigkeit, Formschluss) geschehen. Hier sind beliebige Ausgestaltungen möglich; so kann z. B. auch der Rotor so gestaltet werden, dass der Schaufelblattkörper im Bereich der Nabe gegenüber den Streben gleitet und im nabenfernen Bereich fest mit den Streben verbunden ist. Dies kann auch über die Länge des Rotors variieren.

Die Strebe(n) können wenigstens teilweise aus einem superelastischen Material, insbesondere einem superelastischen Polymer, oder einer Gedächtnislegierung, insbesondere Nitinol, bestehen. Dabei wird unter einem superelastischen Material ein solches verstanden, das wenigstens eine elastisch Dehnung um 2 % übersteht und danach kraftfrei im Wesentlichen in seine Ausgangsposition zurückkehrt.

Auch das Material des Schaufelblattkörpers kann aus einem superelastischen Werkstoff bestehen, beispielsweise um Rissbildung, besonders an den radial äußeren Spitzen des Schaufelblattes und im Bereich der Schaufelblattwurzel an der Nabe zu minimieren bzw. zu verhindern.

Die Strebe bzw. Streben können wenigstens teilweise schräg gegenüber der Axialrichtung der Rotationsachse angeordnet sein, so dass sie zur Axialrichtung einen Winkel von weniger als 90° einnehmen. In diesem Fall kann bei der Kompression in den zweiten Zustand das jeweilige Schaufelblatt nicht nur in Umfangsrichtung an die Nabe angelegt werden, sondern auch teilweise in Richtung der Rotationsachse angeklappt werden. Dies hat den Vorteil, dass eine Kompressionsbewegung teilweise in der Ebene des Schaufelblattes stattfindet, da in dieser Richtung üblicherweise keine Versteifung des Schaufelblattes durch die Streben notwendig ist. Trotz guter Versteifungseigenschaften senkrecht zur Schaufelblattebene können die Streben daher eine begrenzte Bewegung in der Schaufelblattebene zulassen.

Eine weitere vorteilhafte Ausgestaltung der Erfindung sieht vor, dass das Schaufelblatt auf seiner Druckseite wenigstens ein zugfestes Element in Form eines Bandes oder einer Folie aufweist, das an wenigstens einem radial äußeren Befestigungspunkt und wenigstens einem radial inneren Befestigungspunkt mit einer Strebe und/oder dem Schaufelblattkörper verbunden ist. Ein solches zugfestes Element stabilisiert das Schaufelblatt im dritten, gestreckten Zustand, ohne die Kompression in den ersten Zustand merklich zu behindern oder zu erschweren. Das entsprechende zugfeste Element kann entweder direkt in radialer Richtung oder auch schräg zur Rotationsachse, insbesondere parallel oder im Wesentlichen parallel zu den Streben verlaufen.

Es kann zudem vorteilhaft vorgesehen sein, dass der Rotor wenigstens auf der Druckseite des Schaufelblattes wenigstens ein zugfestes Element in Form eines Bandes oder einer Folie aufweist, das einerseits an einer Strebe und/oder dem Schaufelblattkörper in radialem Abstand von der Rotationsachse sowie andererseits an einer Nabe befestigt ist. Das zugfeste Element kann somit am Fuße des jeweiligen Schaufelblattes auch von dessen Wurzel beabstandet an der Nabe befestigt sein. Das zugfeste Element kann auch als Folie die Kontur des Schaufelblattes fortsetzen.

Vorteilhaft kann das zugfeste Element zudem Glasfasern, Polycarbonatfasern oder andere Verstärkungsfasern enthalten. Diese weisen bei geringem Gewicht und Volumen eine sehr hohe Zugfestigkeit und Widerstandsfähigkeit gegen Dehnung auf.

Es kann außerdem vorgesehen sein, dass das zugfeste Element wenigstens teilweise auf der Oberfläche einer Strebe oder des Schaufelblattkörpers haftet. In diesem Fall wird das zugfeste Element zu einer zusätzlichen Schicht der Strebe oder des Schaufelblattkörpers, und es entsteht lokal ein Kompositkörper mit den gewünschten mechanischen Eigenschaften, wie Biegesteifigkeit in einer Belastungsrichtung und gegebenenfalls Biegsamkeit in der entgegengesetzten Richtung.

Effizient kann das zugfeste Element beispielsweise auf die Strebe oder das Schaufelblatt aufgeklebt, aufgespritzt oder aufgedruckt sein.

Das Aufdrucken kann mit allen üblichen Techniken geschehen, die auch zum Aufbringen von optisch erkennbaren Symbolen auf bedruckbare Oberflächen verwendet werden. Zudem ist es möglich, das zugfeste Element durch Schweißen oder Einbrennen mittels eines Lasers aufzubringen.

Das zugfeste Element kann im ersten Zustand des Rotors beispielsweise mäanderförmig gewunden verlaufen. Die entsprechenden Windungen sollten so ausgeprägt sein, dass das zugfeste Element sich im Zuge der Streckung der Schaufelblattes bei der Umformung in den dritten Zustand strecken und damit die weitere Bewegung des Schaufelblattes effektiv begrenzen kann.

Es ist beispielsweise auch denkbar, auf einem Schaufelblatt ein Muster von strahlenförmig auseinanderlaufenden zugfesten Elementen vorzusehen, die beispielsweise von einer gemeinsamen Basis an der Wurzel des Schaufelblattes ausgehend und sich zum radial Äußeren des Schaufelblattes voneinander entfernen.

Es kann auch durch entsprechende Berechnungen der Verlauf der Kraftlinien innerhalb des Schaufelblattes bei Einnahme der gewünschten geometrischen dreidimensionalen Form im dritten Zustand berechnet und durch entsprechende Positionierung der zugfesten Elemente abgebildet werden.

Eine weitere vorteilhafte Ausgestaltung der Erfindung sieht vor, dass das Schaufelblatt schwenkbar in einer mantelseitigen Ausnehmung einer hohlzylindrischen Nabe gelagert ist. Sinn dieser Anordnung ist es, die Materialverformung an der Wurzel des Schaufelblattes bei der Verformung zwischen dem ersten und dem dritten Zustand des Schaufelblattes möglichst zu begrenzen, um Rissbildung und andere Ermüdungserscheinungen des Materials zu vermeiden. Hierzu werden die wirkenden Dehnungskräfte im Bereich der Wurzel des Schaufelblattes minimiert. Entsprechende Haltekräfte auf das Schaufelblatt werden nicht durch Verbindung des Schaufelblattes mit der Nabe erzeugt, sondern durch andere Strukturen. Das Schaufelblatt kann im Bereich der Nabe beispielsweise durch ein Filmgelenk bzw. durch Schwächung des Materials der Nabe gehalten sein.

Die Erfindung kann außerdem vorteilhaft vorsehen, dass das Schaufelblatt mit einem Innenende durch die Öffnung hindurch in den zylindrischen Hohlraum der Nabe hineinragt. Das Schaufelblatt ist dann nach Art eines zweiarmigen Hebels in der Wand des Nabenkörpers gelagert.

Es kann dann vorgesehen sein, dass das Innenende im dritten Zustand mit einem festen Anschlag im Hohlraum der Nabe zusammenwirkt.

Der Hohlraum innerhalb der Nabe kann durch Integrieren eines festen Anschlages derart gestaltet sein, dass eine Bewegung des Innenendes des Schaufelblattes dort nach Erreichen des dritten Zustandes begrenzt wird.

Bei der Kompression des Rotors und des Schaufelblattes findet eine Bewegung in die entgegengesetzte Richtung statt, so dass auch das Innenende des Schaufelblattes sich von dem festen Anschlag im Inneren der Nabe entfernt und möglichst frei bewegt, um die Bewegung des Schaufelblattes nicht zu behindern.

Es kann auch vorgesehen sein, dass wenigstens eine Strebe des Schaufelblattes in den zylindrischen Hohlraum der Nabe hineinragt.

Die Strebe oder Streben des Schaufelblattes können innerhalb des Schaufelblattkörpers oder auch separat von diesem in das Innere der Nabe hineinragen. In letzterem Falle kann der Schaufelblattkörper an der Wand der Nabe enden, und ausschließlich die Streben können in das Innere der hohlzylindrischen Nabe hineinragen. Die einzelnen Streben können dann beispielsweise an ihren in den Hohlraum der Nabe hineinragenden Enden jeweils einen Anschlagkörper aufweisen, der mit einem festen Anschlag der Nabe im dritten Zustand zusammenwirkt.

Ein entsprechender Anschlagkörper an den Enden der Streben kann jeweils als senkrecht zur Rotationsachse liegendes Blechteil ausgebildet sein, das mit der jeweiligen Strebe auch einstückig zusammenhängen kann. Somit können jeweils Strebe und Anschlagkörper einfach zusammenhängend hergestellt werden, z. B. durch Ausstanzen aus einem flachen Körper wie beispielsweise einem Blech.

Der Anschlag kann aber beispielsweise auch durch die Strebe des gegenüberliegenden Schaufelblattes gebildet werden.

Der Anschlag innerhalb der Nabe kann vorteilhaft bei Herstellung der Nabe einstückig und integriert mit dieser geformt sein.

Eine Ausgestaltung der (vorzugsweise superelastischen) Streben ist vorzugsweise derart, dass sowohl bei der Komprimierung als auch im Betriebszustand die zulässige elastische Dehnung in keinem Punkt der Streben überschritten wird (auch lokal). Dies ist bei der geometrischen Gestaltung der Streben, beispielsweise, dass diese mäanderförmig sind, zu berücksichtigen.

Eine Weiterbildung der Erfindung sieht vor, dass das mindestens eine Schaufelblatt eine Strömungsdruckseite und eine Strömungssaugseite aufweist und mindestens eine Versteifungsstrebe so ausgeführt ist, dass bei Erreichen des dritten, expandierten Zustands die Versteifungsstrebe einen mechanischen Anschlag aufweist. Dieser mechanische Anschlag kann auf verschiedene Weisen ausgeführt werden. Beispielsweise ist es möglich, dass die Versteifungsstrebe auf der Strömungssaugseite geschwächt ist. Bei einem radialen Komprimieren des Rotors (in den gelagerten Einführzustand) wird das Komprimieren hierdurch erleichtert, während bei dem Bewegen entgegen dem Fluiddruck (also im Förderbetrieb) eine selbstversteifende Wirkung eintritt.

Dies kann beispielsweise dadurch gewährleistet sein, dass quer zur Steglängsrichtung angebrachte Schlitze die Schwächung darstellen. Im oben genannten dritten Zustand, bei dem der Anschlag gegeben ist, können die Innenwände dieser Schlitze entweder direkt aufeinandergepresst werden oder es kann, falls Kunststoff oder anderes Einbettungsmaterial hier vorhanden ist, eine entsprechende Kompression geschehen, so dass sich hier eine Anschlagssituation ergibt. Wichtig ist lediglich, dass sich der Rotor im Fluid so entfaltet, dass hier ein mechanischer "Anschlag" gegeben ist. Unter "Anschlag" wird eine überproportionale Kraftanstrengung verstanden, welche dem Fluiddruck entgegengesetzt wird; hierdurch ist eine definierte Stellung im dritten Zustand (Betriebszustand) möglich.

Eine weitere vorteilhafte Weiterbildung sieht vor, dass die Versteifungsstrebe, vorzugsweise im radial der Nabe nahen Bereich, eine Versteifungskrümmung aufweist. Dies kann ein "Knie" sein, welches hier zusätzliche Stabilität in die eine Richtung bei gleichzeitig verbesserter Verformbarkeit in entgegen gesetzter Richtung garantiert. Dieses kann im Rahmen fachmännischen Könnens optimiert werden, beispielsweise dahingehend, dass die Dehnung des Bauteils überall weniger als 8 % beträgt. Somit kann bei einem Material beispielsweise bei einem Formgedächtnismaterial, aus dem die Versteifungsstrebe gebildet ist, verhindert werden, dass hier der plastische Bereich der Verformung betreten wird.

Eine weitere vorteilhafte Weiterbildung sieht vor, dass die Versteifungsstrebe im Bereich der Rotorvorderkante, im Bereich der Rotorhinterkante und/oder an einer anderen Stelle des Rotors angebracht ist. Das heißt, dass hier nicht eine Vielzahl von Lamellen axial über die Rotorlänge verteilt angebracht sein muss, sondern dass hier auch punktuelle Versteifungsstreben ausreichen, insbesondere an Stellen, die für die strömungstechnische Form des Rotors wichtig sind.

Eine Weiterbildung sieht vor, dass die Versteifungsstrebe aus einem vorzugsweise ringförmigen Nabenteil sowie einem radialen Ausleger besteht. Vorzugsweise kann das Nabenteil auch formschlüssig mit einer Welle etc. verbunden werden. Außerdem kann insbesondere eine Materialanhäufung im Übergangsbereich vom Nabenteil zum radialen Ausleger verhindert werden, um hier eine noch höhere Elastizität und eine Vermeidung plastischer Verformung zu erreichen; außerdem werden hierdurch elastische Spann-Gegenkräfte bei geeigneter Auslegung zur Verfügung gestellt.

Die Versteifungsstrebe kann prinzipiell aus allen Materialien bestehen, die eine Formgebung bzw. Verformbarkeit günstig beeinflussen können, vorzugsweise Metalle oder Kunststoffe.

Sämtliche Ausführungsformen des Rotors weisen vorzugsweise drei Zustände auf. Dies ist ein erster Zustand, in dem der Rotor kraftfrei ist und die Versteifungsstrebe bzw. das Schaufelblatt radial absteht. In einem zweiten Zustand ist das Schaufelblatt radial komprimiert bzw. legt sich tangential an die Nabe/Achse an.

Im dritten Zustand erfolgt eine Verformung aus dem ersten Zustand heraus durch Fluidgegendruck bzw. Zentrifugalkräfte. Hierbei ist vorzugsweise der Rotor / die Versteifungsstrebe / das Schaufelblatt so auszulegen, dass vom ersten Zustand aus gesehen die Verformung in den zweiten Zustand in einer entgegengesetzten (Dreh-)Richtung erfolgt als bei der Verformung vom ersten in den dritten Zustand.

Dies wird insbesondere bei den oben genannten geschwächten Ausführungsformen der Versteifungsstrebe nützlich sein, da die Schwächung hier dafür sorgt, dass beim Komprimieren besonders geringe Kräfte notwendig sind und andererseits durch die Versteifung / den Anschlag im Betriebszustand (dritten Zustand) hier eine definierte Arbeitsposition gegeben ist, die strömungstechnisch günstig ist und der guten Auslegung des Rotors / einer Pumpe dient.

Es sei nochmals angemerkt, dass sämtliche hier vorgeschlagenen Rotoren/Schaufelblätter insbesondere zum Einsatz in intraventrikulären Blutpumpen geeignet sind. Diese Pumpen weisen einen komprimierten Zustand (zweiter Zustand, s. o.) auf, bei dem der Rotor beispielsweise in eine Schleuse eingebracht ist. In diesem "Einführzustand" wird die Blutpumpe dann in ein Körpergefäß, beispielsweise den linken Ventrikel eines Menschen oder Tieres, eingeführt. Vor dem Pumpbetrieb wird dann der Rotor aus der Schleuse herausgeführt, so dass sich nach Beginn der Drehbewegung dann der dritte Zustand einstellt. Nach Beendigung des Pumpbetriebes erfolgt das erneute Einbringen des Rotors in die Schleuse und die Entnahme der Pumpe.

Im Folgenden wird die Erfindung anhand von Ausführungsbeispielen in einer Zeichnung gezeigt und nachfolgend beschrieben. Dabei zeigt

| | |
|---|---|
| Fig. 1 | schematisch eine Rotorblutpumpe, die mittels eines Hohlkatheters in das linke Ventrikel eines Herzens eingeführt ist, |
| | |
| Fig. 2 | in einer detaillierteren Darstellung die Pumpe, |
| | |
| Fig. 3 | schematisch eine Ausführungsform eines Rotors mit einer Nabe, |
| | |
| Fig. 4 | eine Ausführungsform eines Rotors mit einem nabenlosen Schaufelblatt, |
| | |
| Fig. 5 | die Struktur eines Rotors mit einer Nabe und Verstärkungsstreben für den Schaufelblattkörper, |
| | |
| Fig. 6 | eine Konfiguration von Streben für einen Rotor, wobei die Streben von der Längsachse des Rotors senkrecht abstehen, |
| | |
| Fig. 7 | eine Konfiguration mit Streben, die gegenüber der Rotationsachse des Rotors geneigt sind, |
| | |
| Fig. 8 | einen Querschnitt durch einen Rotor mit einer massiven Nabe, |
| Fig. 9 | einen Querschnitt durch einen Rotor mit einer hohlen Nabe, |
| | |
| Fig. 10 | einen Querschnitt durch einen Rotor mit einer hohlen Nabe und zwei Schaufelblättern, die in Ausnehmungen der Nabe aufgehängt sind, |
| | |
| Fig. 11 | einen Rotor im Querschnitt mit einer hohlen Nabe und Anschlagkörpern, |
| | |
| Fig. 12 | einen Rotor im Querschnitt mit einer hohlen Nabe und in diese hineinragende Streben zweier Schaufelblätter mit entsprechenden Anschlagkonfigurationen, |
| | |
| Fig. 13 | im Querschnitt einen Rotor mit zwei Schaufelblättern und darin gebetteten Streben, |
| | |
| Fig. 14 | im Teilquerschnitt einen Rotor mit zwei Schaufelblättern, in die Streben innerhalb von Hohlräumen integriert sind, |
| | |
| Fig. 15 | im Querschnitt einen Rotor mit einem Schaufelblatt und einer darin integrierten Strebe in einem Hohlraum, der länger ist als die Strebe, |
| | |
| Fig. 16 | eine dreidimensionale Außenansicht eines Rotors mit Schaufelblättern und zugfesten Elementen zur Stützung der Schaufelblätter sowie |
| Fig. 17 | einen Rotor mit einer Mehrzahl von Schaufelblättern und verschiedenartigen, diese stützenden Zugelementen. |
| | |
| Fig. 18 bis 20 | eine Ausführungsform einer Verstärkungsstrebe in verschiedenen Zuständen, wobei die Strebe auf der Strömungssaugseite Ausnehmungen zur besseren Komprimierbarkeit aufweist, wobei die Ausnehmungen im Betriebszustand weitgehend geschlossen sind und zusammen mit dem eingebetteten Grundwerkstoff einen Anschlag bilden |
| | |
| Fig. 21 bis 23 | eine Ausführungsform einer Verstärkungsstrebe in verschiedenen Zuständen, wobei die Strebe hinsichtlich gleichmäßiger Spannungsverteilungen im Werkstoff optimiert ist |
| | |
| Fig. 24 bis 26 | eine Ausführungsform einer Verstärkungsstrebe in verschiedenen Zuständen, wobei die Strebe und ein flexibles Zugelement einteilig ausgebildet sind. |

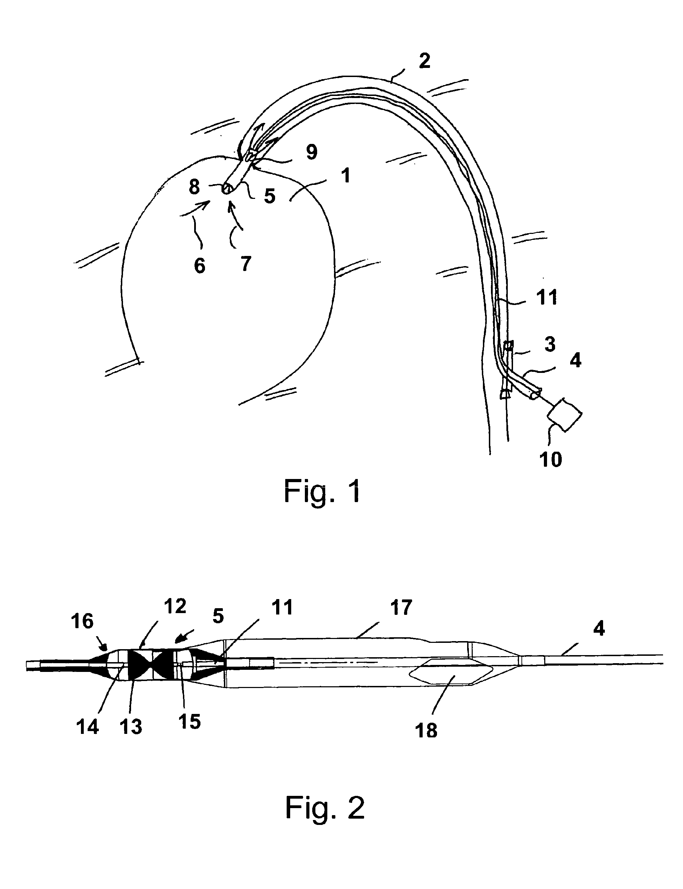

Fig. 1 zeigt schematisch das linke Ventrikel 1 einer Herzkammer 1 sowie ein in dieses mündendes Blutgefäß 2, in das mittels einer Schleuse 3 ein Hohlkatheter 4 eingeführt ist. Dieser hält an seinem distalen Ende eine Pumpe 5, die wenigstens teilweise in das Ventrikel 1 hineinragt. Die Pumpe 5 weist an ihrem distalen Ende einen Ansaugkäfig 8 auf, in den, wie durch die Pfeile 6, 7 angedeutet, Blut angesaugt wird. Dieses wird durch Abstromöffnungen 9 innerhalb des Blutgefäßes 2 hinter der Herzklappe ausgepresst. Die Pumpe 5 weist einen Rotor mit Förderelementen auf, der um seine Längsachse rotierend mittels einer flexiblen Welle 11 antreibbar ist , die durch den Hohlkatheter 4 verläuft und außerhalb des Körpers des Patienten mit einem Antriebsmotor 10 verbunden ist. Die typische Drehzahl des Rotors liegt im Betrieb bei einigen tausend bis ca. 50 000 U/min.

In der Fig. 2 ist detaillierter die Pumpe 5 mit dem Rotor 13 dargestellt. Innerhalb eines Pumpengehäuses 12 ist der Rotor 13 mittels einer Rotorwelle 14 am proximalen Ende 15, d. h. dem der Schleuse 3 näheren Ende, sowie am distalen Ende 16 jeweils drehbar gelagert. Jenseits der Lagerung am distalen Ende 16 oder zwischen dem distalen Ende und dem Rotor 13 selbst sind Ansaugöffnungen für das Blut vorgesehen, beispielsweise in Form eines Ansaugkäfigs, d. h. einer Durchbrechung des Gehäuses 12.

Der Rotor 13 selbst ist einteilig ausgeführt, mit einer Nabe und zwei mit dieser einstückig verbundenen helixförmigen Schaufelblättern.

An dem Pumpengehäuse 12 setzt ein Abströmschlauch 17 an, der bei richtiger Positionierung der Pumpe innerhalb der Herzklappe beim Übergang zwischen dem Blutgefäß 2 und dem Ventrikel 1 positioniert wird, so dass die Abströmöffnungen 18 innerhalb des Blutgefäßes 2 liegen. Am distalen Ende der Pumpe 5 ist ein zusätzliches abstandhaltendes Teil, das am freien Ende abgebogen, beispielsweise spiralig geformt ist, um das Anstoßen der Pumpe an Körpergewebe zu verhindern und zudem das Einschieben der Pumpe durch ein Blutgefäß zu erleichtern. Außerdem soll das Element verhindern, dass sich die Pumpe an Gefäßwänden oder den Innenwänden einer Herzkammer festsaugt.

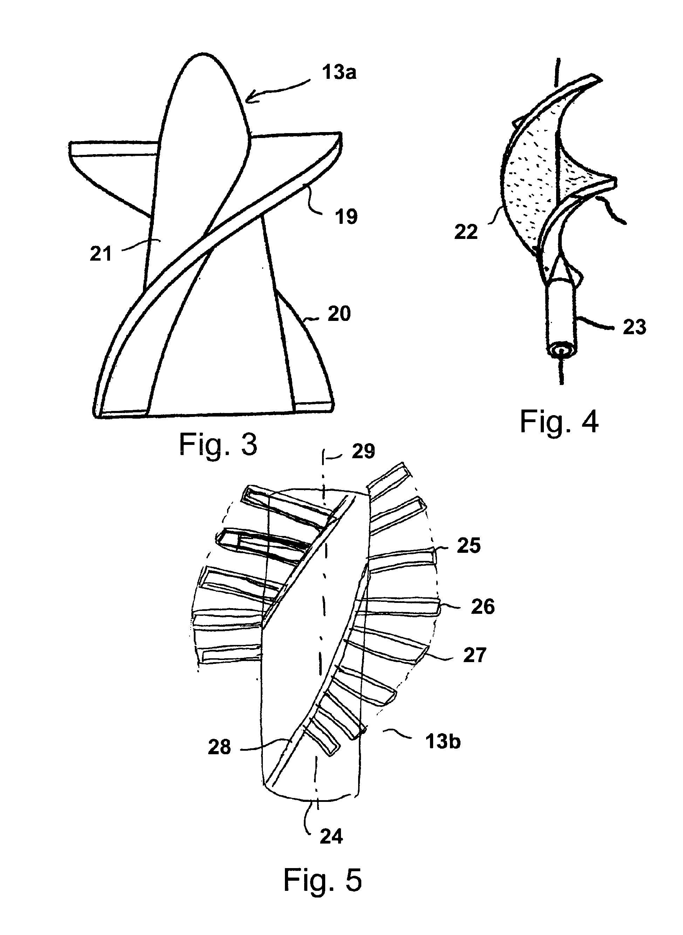

Fig. 3 zeigt einen Rotor 13a mit zwei helixförmigen Schaufelblättern 19, 20, die aus Kunststoff bestehen, mit der Nabe 21 einteilig ausgebildet sind und beispielsweise Stützstreben in ihrem Inneren aufnehmen können. Der Schaufelblattkörper der Schaufelblätter 19, 20 kann beispielsweise aus Polyurethan in massiver Form oder aus einem Schaumstoff bestehen, und in den Körper können die Streben integriert sein. Die Herstellung kann dadurch erfolgen, dass die Streben mit einem entsprechenden Kunststoff umspritzt werden.

Fig. 4 zeigt schematisch in dreidimensionaler Ansicht einen Rotor ohne eine Nabe mit einem einzigen Schaufelblatt 22. Auch in dieses können entsprechende Streben eingebettet sein. Das Schaufelblatt 22 wird stirnseitig über ein Wellenstück 23 angetrieben.

Fig. 5 zeigt einen Rotor 13b mit einer Nabe 24 sowie zwei Reihen von Streben 25, 26, 27, die jeweils in Form einer Helix umlaufend an der Nabe 24 verteilt sind und von dieser radial abstehen. Die Streben 25, 26, 27 können beispielsweise jeweils aus einem Paar von zwei Einzelstreben bestehen, die an ihrem rotationsachsfernen Ende miteinander verbunden sind. Die Rotationsachse ist in der Fig. 5 mit 29 bezeichnet.

Auf diese Weise werden durch die Streben 25, 26, 27 Schlaufen gebildet, die sich gut in einem Schaufelblattkörper verankern lassen.

Die Streben können beispielsweise gemeinsam auf je einer Leiste pro Schaufelblatt befestigt sein, wobei die Leiste flexibel ist und sich in eine Ausnehmung 28, die an der Nabe 24 umläuft, einziehen lassen lässt.

Somit sind bei dem genannten Beispiel jeweils direkt benachbarte Streben paarweise miteinander verbunden, und die Paare sind in ihrem rotationsachsnahen Bereich miteinander verbunden.

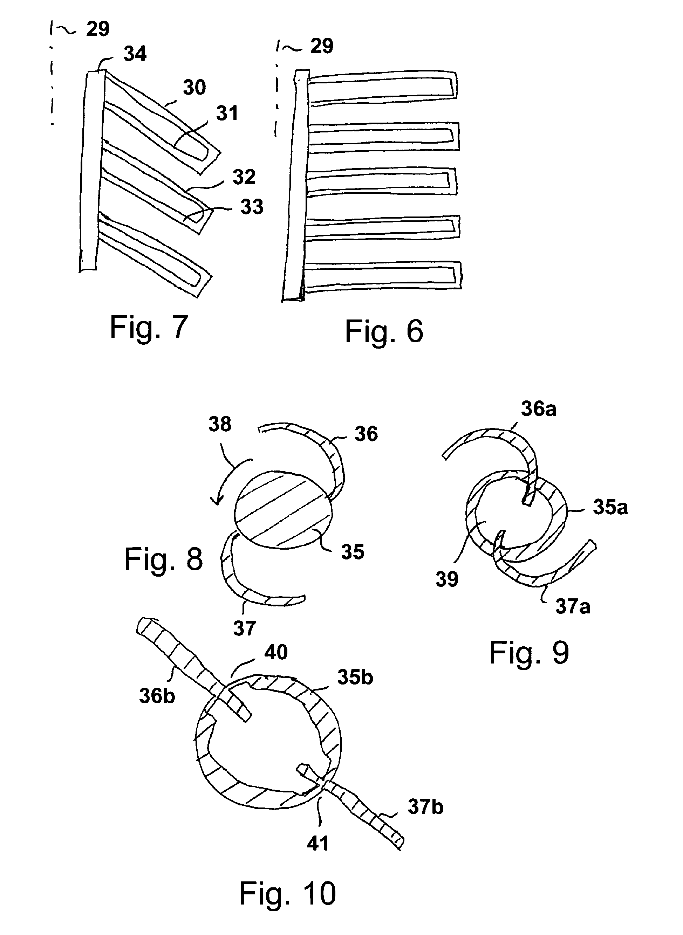

Fig. 7 zeigt Streben 30, 31, 32, 33, die paarweise jeweils in ihrem der Rotationsachse 29 fernen Bereich miteinander verbunden und mittels einer durchgehenden Leiste 34 im rotationsachsnahen Bereich verbunden sind. Die einzelnen Streben 30, 31, 32, 33 verlaufen gegenüber der Rotationsachse 29 geneigt, beispielsweise in einem Winkel von etwa 30° oder 60°. Im Gegensatz dazu zeigt die Fig. 6 Streben, die von der Rotationsachse 29 radial senkrecht abstehen.

Fig. 8 zeigt einen Querschnitt eines Rotors mit einer massiven Nabe 35, an deren mantelseitiger Oberfläche zwei Schaufelblätter 36, 37 im zweiten Zustand, d. h. in leicht vorgekrümmter Form, befindlich dargestellt sind. Im Betrieb, d. h. bei Rotation des Rotors in Richtung des Pfeiles 38, richten sich die Schaufelblätter 36, 37 weiter bis fast zur Streckung auf.

Der Rotor kann auch gegenüber der in Fig. 8 dargestellten Konfiguration weiter komprimiert werden, indem die Schaufelblätter an die Nabe 35 enger angedrückt werden.

Fig. 9 zeigt zwei Schaufelblätter 36a, 37a, die in einen Hohlraum 39 einer Nabe 35a hineinragen. Dadurch bilden die Schaufelblätter 36a, 37a zweiarmige Hebel, die jeweils in der Wand der hohlen Nabe 35a gelagert sind.

Fig. 10 zeigt eine Weiterentwicklung der Ausgestaltung aus Fig. 9, indem bei der Nabe 35b die Wandstärke im Bereich 40, 41, wo die Schaufelblätter 36b, 37b die Wand der Nabe 35b durchdringen, jeweils durch Ausdünnung geschwächt ist. Dadurch sind die Schaufelblätter in einer Art Filmgelenken in entsprechenden Bereichen der Nabe schwenkbar gelagert.

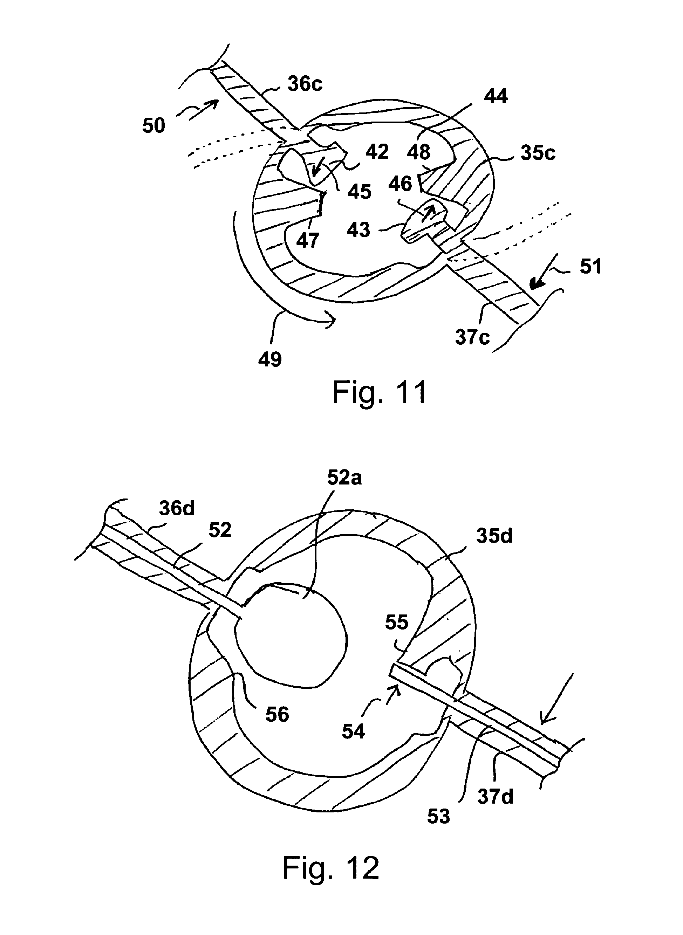

Fig. 11 zeigt eine entsprechende Konstellation, bei der zwei Schaufelblätter 36c, 37c jeweils mit ihren Innenenden und daran befestigten Anschlagkörpern 42, 43 in den Hohlraum 44 einer Nabe 35c hineinragen. Im Innenraum der Nabe wirken die Anschlagkörper 42, 43 in Richtung der Pfeile 45, 46 im Betrieb des Rotors mit entsprechenden Anschlägen 47, 48 zusammen, so dass die Schaufelblätter 36c, 37c im dritten Zustand gestützt werden und damit den gewünschten Betriebszustand einnehmen.

Bei der Kompressionsbewegung lösen sich die Anschlagkörper 42, 43 von den Anschlägen 47, 48, und die Schaufelblätter 36c, 37c können in die in der Fig. 11 gestrichelt eingezeichneten Positionen gebracht werden. Dabei werden die Schaufelblätter innerhalb der entsprechenden geschwächten Bereiche des Nabenkörpers 35c geschwenkt.

Der Rotationssinn des dargestellten Rotors im Betrieb ist in der Fig. 11 durch den Pfeil 49 dargestellt, während der Fluidgegendruck auf die Schaufelblätter durch die Pfeile 50, 51 symbolisiert ist.

Fig. 12 zeigt eine weitere Ausgestaltung eines Rotors mit einem Nabenkörper 35d und zwei Schaufelblätter 36d, 37d, die jeweils die Streben 52, 53 aufweisen. In dem Ausführungsbeispiel enden die Schaufelblätter an der äußeren Mantelfläche der Nabe 35d, und ausschließlich die Streben 52, 53 ragen in das Innere der Nabe. Dort schlägt die Strebe 53 in Richtung des Pfeils 54 an einen Anschlag 55 im Innenraum der Nabe an, wenn der dritte Zustand des Rotors erreicht ist. Dadurch wird das entsprechende Schaufelblatt 37d gestützt.

Gegenüberliegend ist eine andere Konfiguration anhand des Schaufelblattes 36d dargestellt. Die Darstellung verschiedener Konfigurationen von Schaufelblättern an einem Rotor geschieht hier nur beispielhaft.

Die Strebe 52 des Schaufelblattes 36d läuft im Inneren der Nabe 35d in einem kreisscheibenförmigen Anschlagkörper 52a aus, der mit der Strebe 52 einteilig ausgeführt, beispielsweise aus einem Blech ausgestanzt sein kann. Der Anschlagkörper 52a ist derart geformt, dass er bei entsprechender Belastung im dritten Zustand an einem Anschlag 56, der in diesem Fall keine spezielle Gestaltung benötigt, zur Begrenzung der Bewegung des Schaufelblattes 36d anschlägt.

Innerhalb eines Rotors können auch mehrere der Anschlagkörper 52a miteinander verbunden sein, um ein Gerüst für die Streben 52 zu bilden und diese zu halten, während sie beispielsweise mit dem Material des Schaufelblattkörpers umspritzt werden.

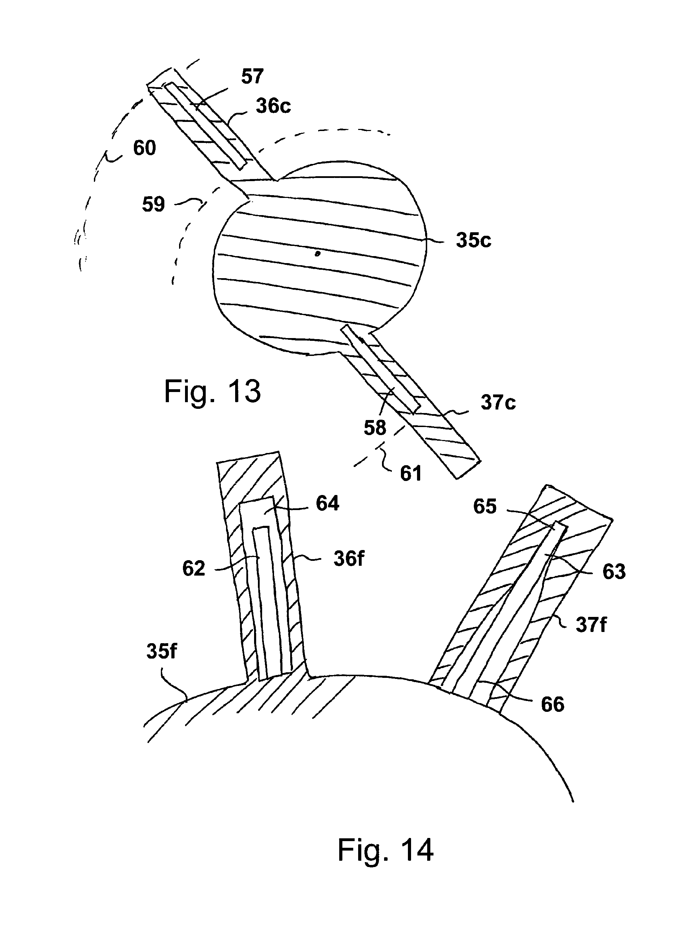

Fig. 13 zeigt einen Rotor mit einer Nabe 35e in massiver Bauweise, mit zwei Schaufelblättern 36e, 37e, in die jeweils Streben 57, 58 integriert sind. Die Strebe 57 reicht von einem ersten Achsabstand, dargestellt durch die gestrichelte Linie 59, bis zu einem zweiten Achsabstand, dargestellt durch die Linie 60. Die Strebe 57 reicht demnach nicht bis zur Nabe 35e.

Die Strebe 58 reicht bis in die Nabe 35e hinein und endet an dem Achsabstand 61, d. h., sie ist radial in Bezug auf die Rotorachse kürzer als das Schaufelblatt 37e. Die Unterschiede der Streben 57, 58 sind nur beispielhaft an einem einzigen Rotor dargestellt.

In den genannten Beispielen sind die Streben jeweils mit dem Material der Schaufelblattkörper umspritzt und haften teilweise oder allseits an dem Material.

In der Fig. 14 ist eine Nabe 35f mit zwei beispielhaften Schaufelblättern 36f, 37f dargestellt, die jeweils eine Strebe 62, 63 aufnehmen. Die Strebe 62 ist in Bezug auf die Länge und die Breite kleiner als der Hohlraum 64, in dem sie sich innerhalb des Schaufelblattes 36f befindet. Die Strebe 63 ist an ihrem radial äußeren Ende 65 fest vom Material des Schaufelblattes 37f umschlossen und kann sich im rotationsachsnahen Bereich 66 innerhalb des Hohlraums des Schaufelblattes 37f bewegen und dort je nach dem Kompressions- oder Expansionszustand verschiedene Positionen einnehmen, d. h. sich an verschiedene Wände des Hohlraums anlegen. Dieser Hohlraum kann asymmetrisch gestaltet sein.

Durch diese Konstellation wird ermöglicht, dass in einer Position, beispielsweise im dritten Zustand des Rotors, die entsprechende Strebe 63 das Schaufelblatt 37f stützen kann, während der Rotor ohne eine Stützwirkung der Strebe stark komprimiert werden kann, indem die Strebe innerhalb des Schaufelblattes ausweichen kann. Entsprechend kann auch vorgesehen sein, dass die Strebe in dem Schaufelblatt nur an ihrem achsnahen Ende fest umschlossen und fixiert ist und im achsfernen Bereich Bewegungsspielraum hat.

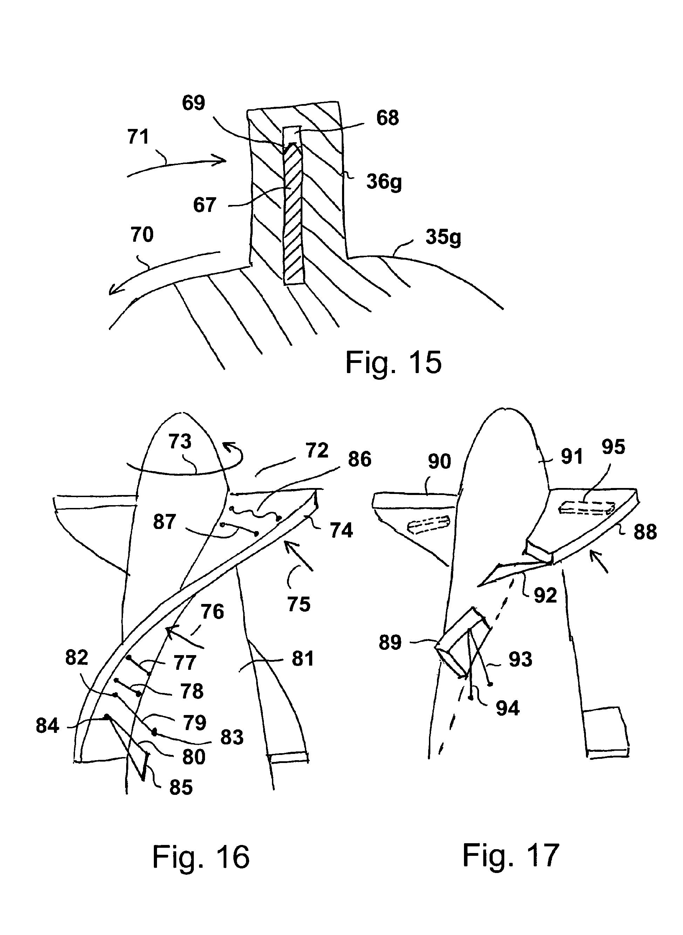

In der Fig. 15 ist ein Rotor mit einer Nabe 35g sowie einem Schaufelblatt 36g mit einer in dieses integrierten Strebe 67 dargestellt. Die Strebe 67 ist in radialer Richtung kürzer ausgeführt als der Hohlraum 68 innerhalb des Schaufelblattes 36g, so dass im Betrieb beispielsweise durch Zentrifugalkräfte die Strebe 37 radial nach außen rutschen und dort das Schaufelblatt stützen kann. Es kann dann bei einer Kompressionsbewegung, beispielsweise durch entsprechende Schrägen am radial äußeren Ende der Strebe, diese radial nach innen verschoben werden, so dass sie eine Kompression weniger behindert als im radial äußeren Bereich. Die Schrägen sind am radial äußeren Ende der Strebe 67 dargestellt und mit 69 bezeichnet. Die Drehbewegung des Rotors ist durch den Pfeil 70 symbolisiert und der Fluidgegendruck gegen das Schaufelblatt 36g durch den Pfeil 71.

Die Fig. 16 zeigt in dreidimensionaler Ansicht einen Rotor 72, der im Betrieb in Richtung des Pfeiles 73 rotiert. Entsprechend ergeben sich an den Schaufelblättern 74 Kräfte in Folge des Fluidgegendrucks, die durch die Pfeile 75, 76 dargestellt sind.

In der Figur sind Zugelemente 77, 78, 79, 80 dargestellt, die unterschiedliche Beispiele für ihre Ausrichtung und Befestigung repräsentieren. Die Zugelemente 77, 78 beispielsweise verlaufen von einem radial äußeren Punkt an dem Schaufelblatt 74 zu einem radial inneren Punkt nahe der Nabe 81. Das Zugelement 79 ist einerseits an einem Punkt 82 an dem Schaufelblatt 74 befestigt, andererseits am Punkt 83 auf der Oberfläche der Nabe 81 in einer Entfernung vom Fuß des Schaufelblatts. Das Zugelement 80 ist flächenhaft als dünne Folie ausgebildet und an einem Punkt 84 am Schaufelblatt befestigt, während die Basis 85 auf der Oberfläche der Nabe 81 in Abstand vom Fuß des Schaufelblatts 74 befestigt ist. Die Folie 80 kann derart ausgerichtet sein, dass sie die Fluidströmung nicht behindert.

Im komprimierten zweiten Zustand oder im ersten Zustand des Rotors sind die entsprechenden Zugelemente schlaff und behindern eine Bewegung der Schaufelblätter 74 nicht. Im dritten Zustand, wenn die Schaufelblätter gestreckt sind, begrenzen die Zugelemente eine weitere Überstreckung und stabilisieren damit die Schaufelblätter.

Die Zugelemente können beispielsweise aus einem Band mit Glasfaserverstärkung oder im Wesentlichen nur aus Glasfasern oder auch aus Polycarbonatfasern, beispielsweise aus Kevlar, bestehen, um eine möglichst geringe Dicke bei entsprechend hoher Zugkraft und Festigkeit zu erzielen. Die Zugelemente können aber auch aus einer Polymerfolie, beispielsweise aus PEEK aber auch aus einer Metallfolie, beispielsweise aus Nitinol oder Titan, bestehen, um den gewünschten Effekt zu erzielen. Prinzipiell sind für diese flexiblen Zugelemente solche Werkstoffe bzw. Werkstoffkombinationen besonders geeignet, die auch für sogenannte non-compliant Ballonkatheter verwendet werden, da diese üblicherweise die hier gewünschten mechanischen Eigenschaften mit den ebenfalls geforderten biokompatiblen und blutverträglichen Eigenschaften verbinden.

An dem Schaufelblatt 74 sind zudem zwei Zugelemente 86, 87 eingezeichnet, wobei das Zugelement 86 auf die Oberfläche des Schaufelblattes 74 aufgeklebt, aufgedruckt oder auf andere Weise befestigt ist und eine gewundene Form aufweist, die nur im ersten oder zweiten Zustand des Schaufelblattes in dieser Form vorliegt. Wird das Schaufelblatt in den dritten Zustand gestreckt, so nimmt dieses Zugelement die mit 87 bezeichnete gestreckte Form an, um dann straff zu sein und eine weitere Bewegung des Schaufelblattes über den gestreckten Zustand hinaus zu verhindern.

In der Fig. 17 ist ein Rotor mit einer Mehrzahl von Schaufelblättern 88, 89, 90 dargestellt, die jeweils nur teilweise um die Nabe 91 umlaufen und einzeln mittels Zugelementen 92, 93, 94 gestützt sein können. Die Zugelemente 92 können als Folien ausgebildet sein und die Kontur der entsprechenden Schaufelblätter 88 hydrodynamisch günstig fortsetzen. Sie sind so positioniert und gespannt, dass sie die Schaufelblätter im gestreckten Zustand stützen. Zudem können die Schaufelblätter 88, 89, 90 in sich Streben 95 integriert haben.

Die genannten Varianten bei der Ausführung der Erfindung stellen einzeln und in Kombination miteinander effiziente Maßnahmen zur Stützung von als Kompositkörper aufgebauten Schaufelblättern an einem Rotor einer Pumpe dar.

Die entsprechenden Rotoren können jeweils in einem komprimierbaren und expandierbaren Gehäuse angeordnet sein, das beispielsweise mittels der sich aufrichtenden Schaufelblätter expandierbar ist.

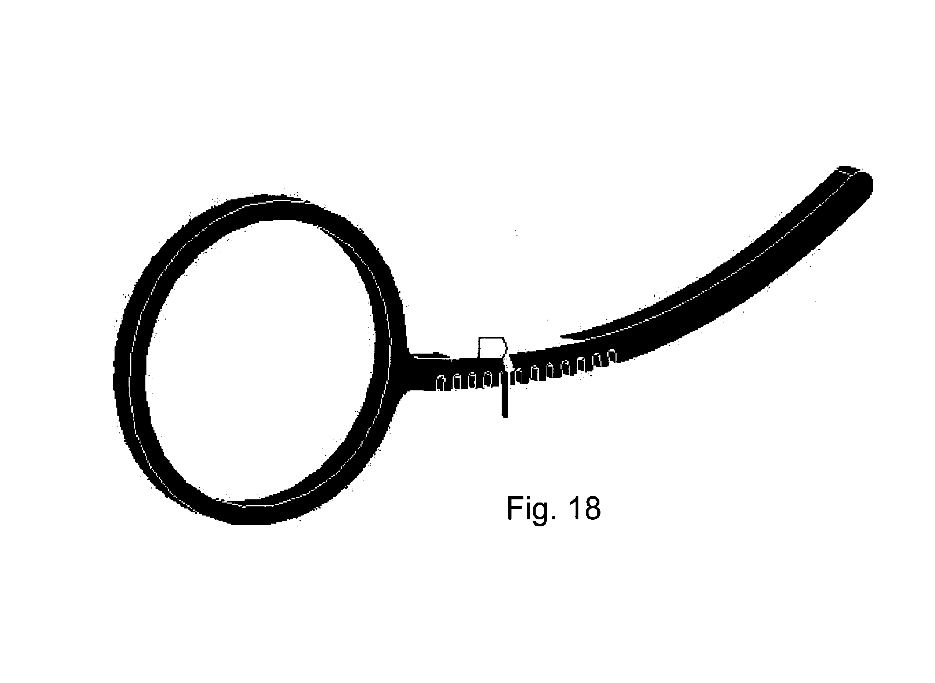

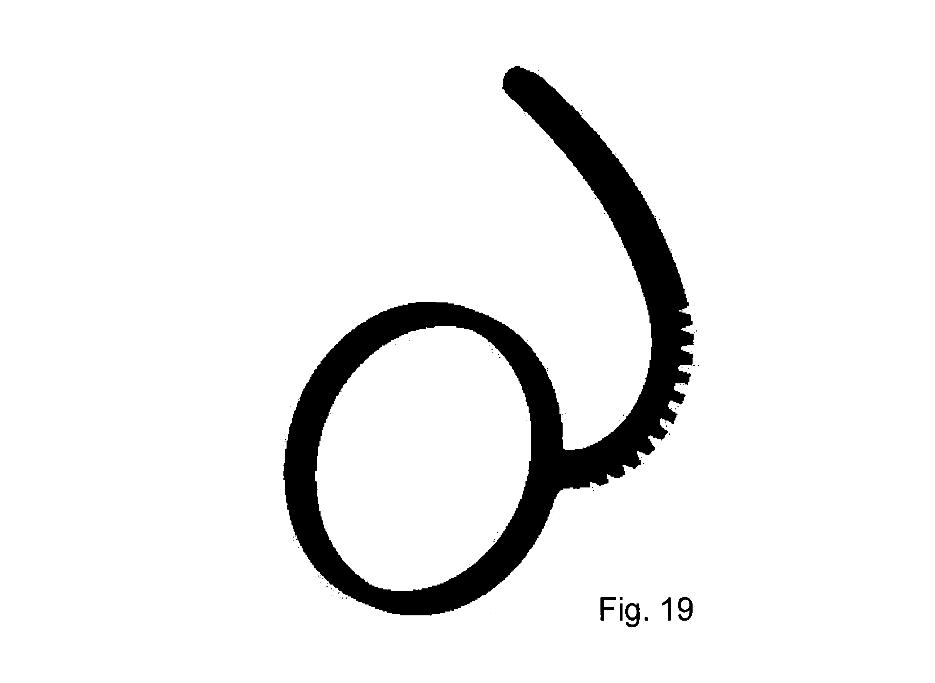

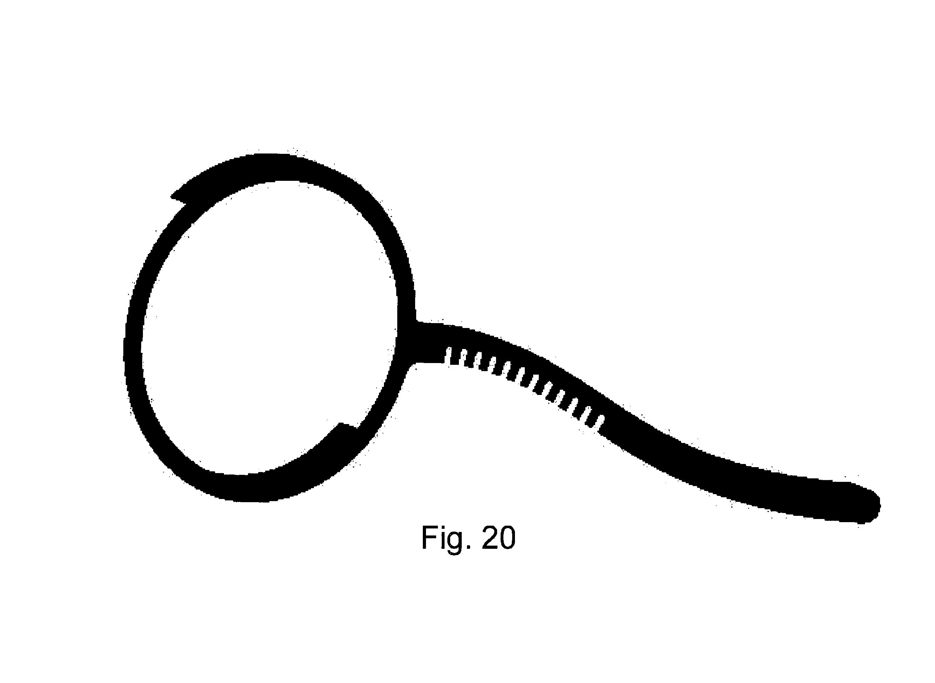

Die Figuren 18 bis 20 zeigen eine weitere Ausführungsform einer Versteifungsstrebe. Hierbei ist in Fig. 18 der erste Zustand gezeigt, also der kraftfreie Zustand. In Fig. 19 ist der zweite Zustand, also der Zustand, in dem die Versteifungsstrebe / das Schaufelblatt / der Rotor radial komprimiert ist. Dies erfolgt vorliegend primär durch ein Kippen der Versteifungsstrebe in Fig. 19 nach links, d. h. entgegen dem Uhrzeigersinn. In Fig. 20 ist der dritte Zustand, also der Betriebszustand des Rotors / des Schaufelblatts / der Versteifungsstrebe gezeigt, bei dem tatsächlich Fluid gefördert wird. Hierbei wirkt der Fluidgegendruck im Uhrzeigersinn, d. h., die Drehrichtung des Rotors ist entgegen dem Uhrzeigersinn. Hierbei ist eine Schwächung auf der Strömungssaugseite (in Fig. 20 an dem radialen Ausleger der Versteifungsstrebe unten zu sehen) gezeigt. Die Strömungsdruckseite ist in diesem Fall ungeschwächt.

In den Figuren 18 bis 20 ist lediglich die Versteifungsstrebe gezeigt, ohne zusätzliches Material, in das die Versteifungsstrebe eingebettet ist. Vorliegend ist diese Versteifungsstrebe an der axialen Strömungsvorderkante des Rotors angebracht, und der in Fig. 18 nach rechts abstehende radiale Ausleger der Versteifungsstrebe ist vollkommen von dem Einbettungsmaterial, vorliegend Kunststoff, umgeben. Dies ist auch zwischen den Schwächungsschlitzen der Fall. Die Schaufelblattoberfläche ist hierbei so geschaffen, dass keine Schwächungen von außen sichtbar sind. Außerdem ist zu beachten, dass das Komprimieren (siehe Fig. 19) besonders leicht ist, während bei dem in Fig. 20 gezeigten Zustand ein "mechanischer Anschlag" gegeben ist. Hierbei müssen nicht die Innenwände der Schlitze gegeneinanderschlagen, da die in diesem Zwischenbereich befindliche Einbettungsmasse/Einbettungsmaterial/Kunststoff komprimiert wird und somit eine Verformungsbegrenzung garantiert.

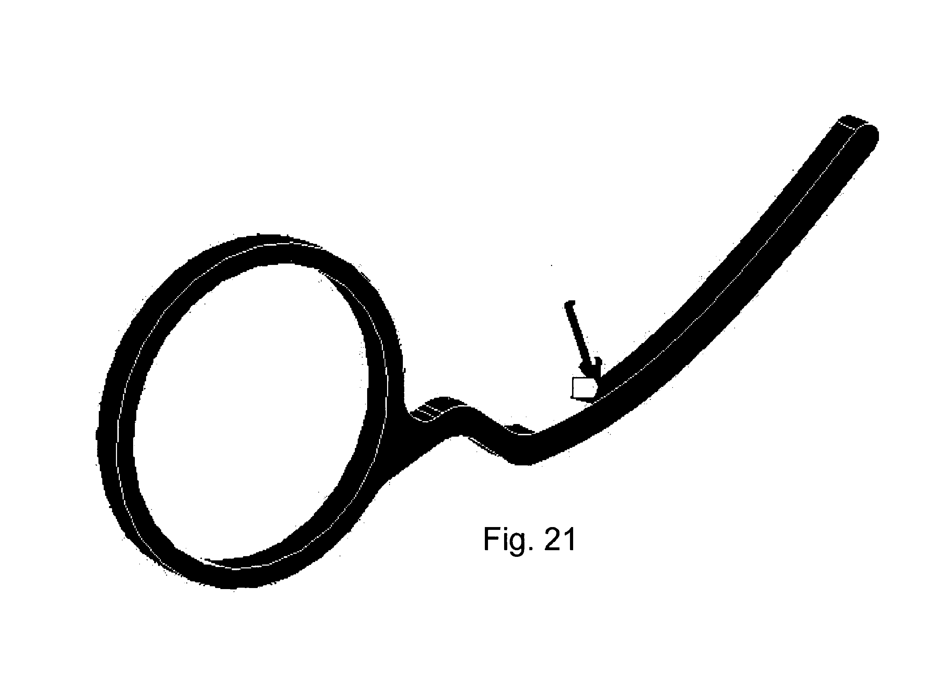

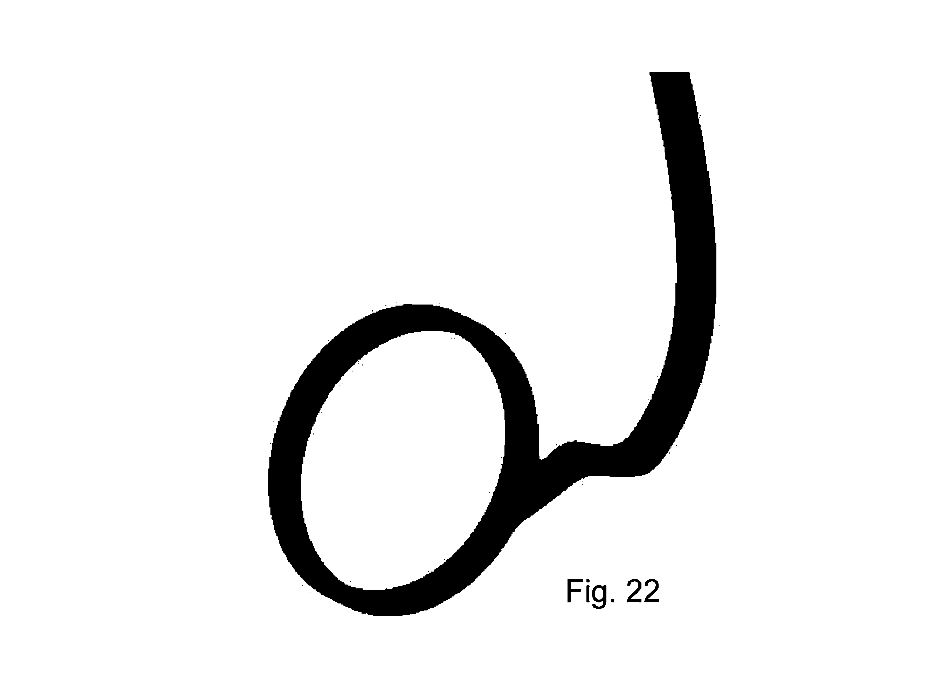

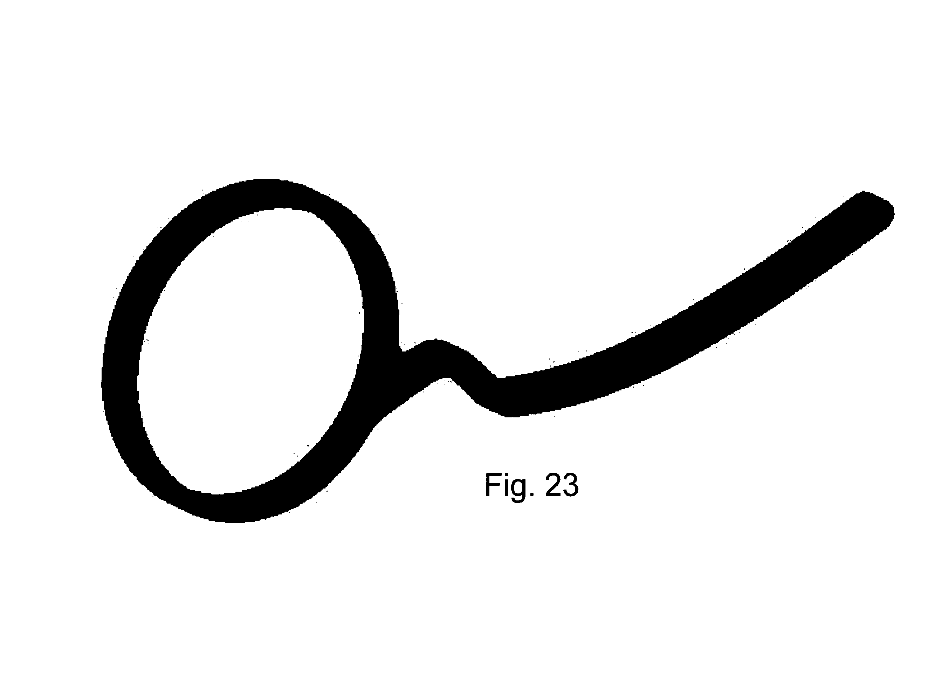

Fig. 21 zeigt wiederum den ersten Zustand, in dem die Verformungsstrebe / der Rotor kraftfrei absteht. Sämtliche Merkmale sind wie bei der vorgenannten Ausführungsform nach Fign. 18-20 ausgebildet, sofern im Folgenden nichts anderes gesagt wird. Bei den Ausführungsformen nach den Fign. 21-23 (Fig. 22 zeigt den zweiten Zustand, Fig. 23 wiederum den dritten Zustand) ist keine Schwächung in Form von Schlitzen gezeigt, stattdessen ist, nahe dem kreisförmigen Nabenbereich, ein Knick gezeigt, der eine "Verstärkungskrümmung" darstellt. Diese ist so ausgelegt, dass einerseits beim elastischen Verformen in den in Fig. 22 gezeigten Zustand ein leichtes Umbiegen möglich ist und andererseits beim Zustand nach Fig. 23 ein mechanischer Anschlag gegeben ist, ohne dass bei dem Material jemals der plastische Bereich betreten wird, bei dem irreversible Verformungen entstehen würden.

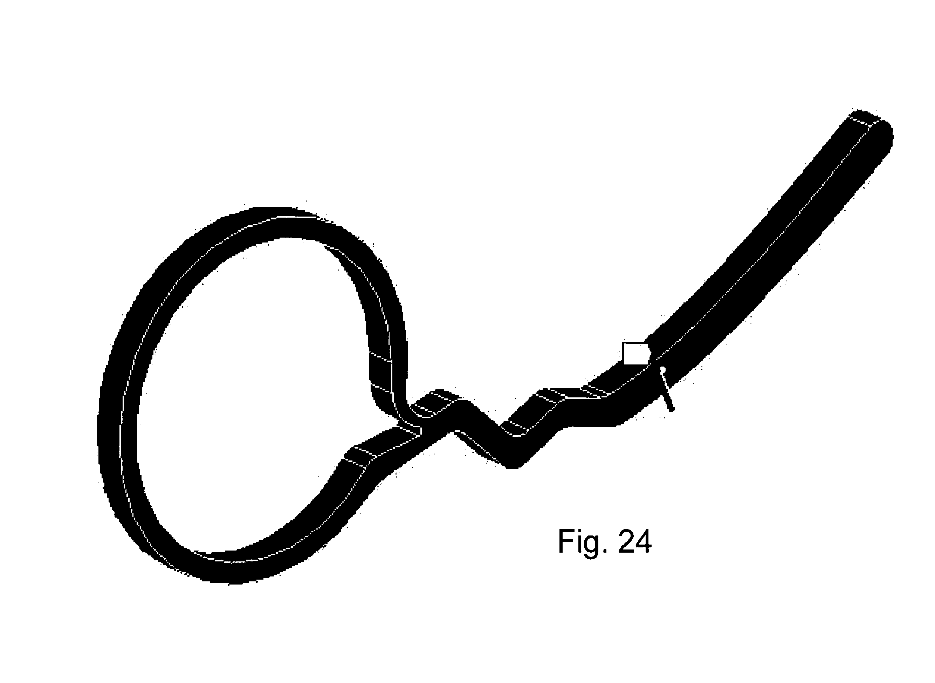

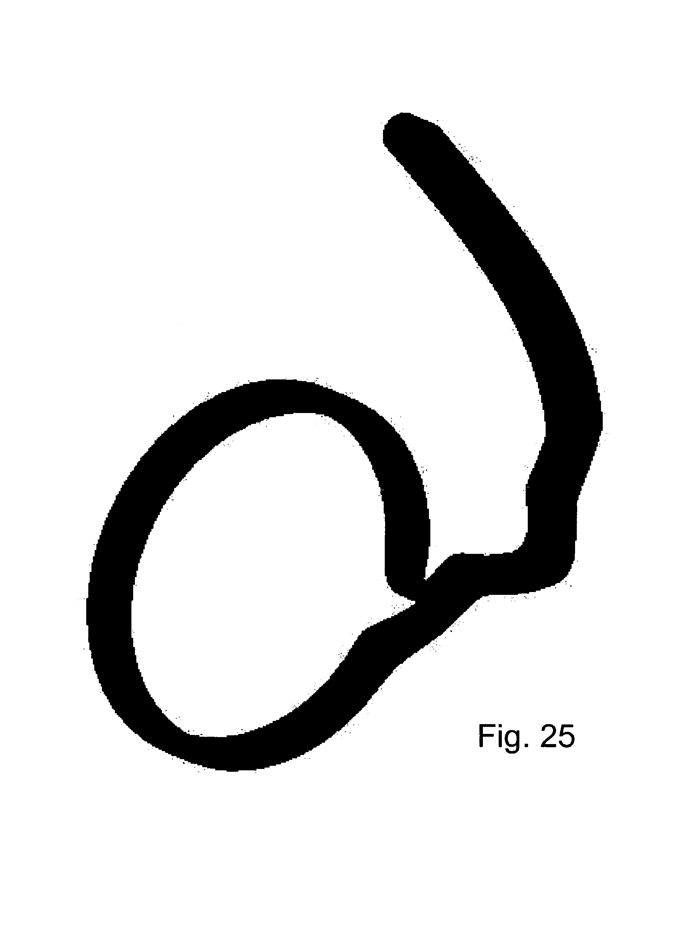

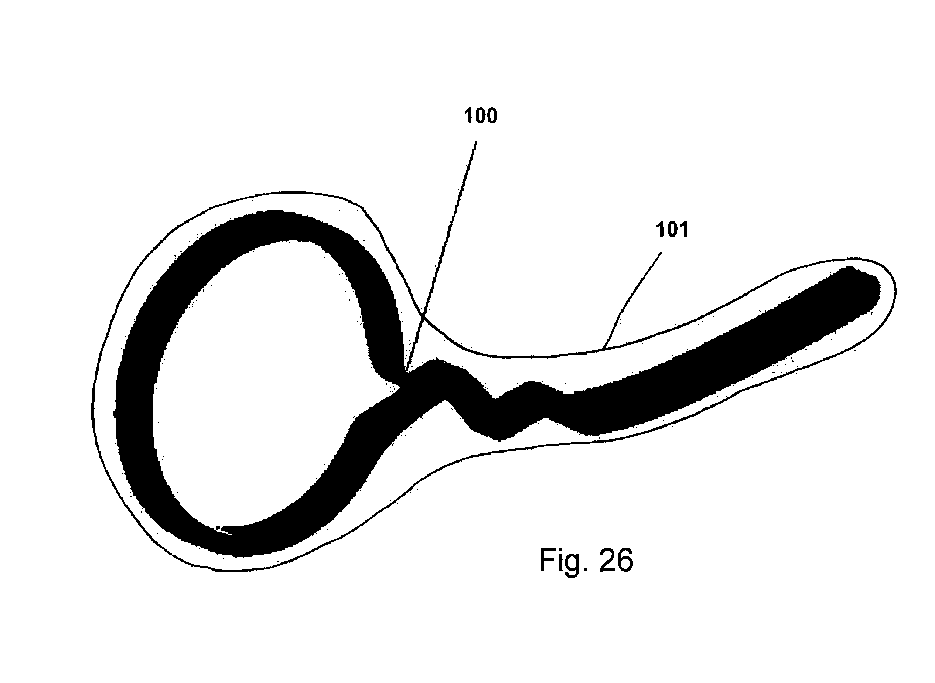

Die Figuren 24 bis 26 zeigen eine weitere Ausführungsform, welche der Ausführungsform nach Fign. 21-23 ähnlich ist, wobei allerdings im Übergangsbereich vom ringförmigen Nabenbereich zum radialen Ausleger hin eine radial innen im Nabenring befindliche Schwächung gezeigt ist, welche die Elastizität des Auslegers, vor allem beim Bewegen vom in Fig. 24 gezeigten ersten Zustand in den in Fig. 25 gezeigten zweiten Zustand erleichtert. Außerdem wird durch eine geeignete Konfiguration auch die Gewährleistung des mechanischen Anschlags in dem in Fig. 26 gezeigten dritten Zustand gewährleistet, der beispielsweise durch die Auslegung des verjüngten Bereichs (100) als Zugstrebe erfolgen kann. Der verjüngte Bereich (100) ist dadurch bei der Einnahme des Ersten Zustands gut biegbar, nimmt aber im dritten Zustand eine annähernd gestreckte Form ein, die einem weiteren Ausweichen gegen den Strömungsdruck einen erhöhten Widerstand entgegensetzt.
Es sei nochmals erwähnt, dass in den Figuren 20, 23 und 26 jeweils angenommen wird, dass die Drehrichtung des Rotors entgegen dem Uhrzeigersinn ist und sich dadurch ein Aufdehnen des radialen Auslegers in Richtung des Uhrzeigersinnes ergibt.
In Fig. 26 ist weiterhin schematisch der Umriss eines Schaufelblattes 101 dargestellt, welches in der dargestellten Ausführungsform die Strebe vollständig derart umschließt, dass das Fluid mit einer möglichst gleichmäßigen homogenen Oberfläche in Berührung kommt. Dies gewährleistet, dass das Fluid im Betriebszustand eine möglichst gleichmäßige Beschleunigung mit geringen Scherspannungsspitzen erfährt.

Aspekte der Erfindung sind unter anderem:
1. Radial komprimierbarer und expandierbarer Rotor (13, 13a, 13b) für eine Pumpe mit wenigstens einem Schaufelblatt (19, 20, 22, 36, 36a, 36b, 36c, 36d, 36e, 36f, 36g, 37, 37a, 37b, 37c, 37d, 37e, 37f, 74, 88, 89, 90), wobei das Schaufelblatt einen Schaufelblattkörper aufweist, dessen Material elastisch verformbar ist, sowie wenigstens eine Versteifungsstrebe (25, 26, 27, 30, 31, 32, 52, 53, 57, 58, 62, 63, 67, 95), die in das Material des Schaufelblattkörpers wenigstens teilweise eingebettet ist.
2. Rotor nach Aspekt 1, dadurch gekennzeichnet, dass dieser ohne äußere Krafteinwirkung einen ersten Zustand einnimmt, von dem ausgehend er durch radiale Kompression in einen zweiten Zustand überführbar ist.
3. Rotor nach Aspekt 1 oder 2, dadurch gekennzeichnet, dass dieser ohne äußere Krafteinwirkung einen ersten Zustand einnimmt, von dem ausgehend das wenigstens eine Schaufelblatt im Betrieb insbesondere durch den auftretenden Fluidgegendruck und/oder Zentrifugalkräfte bei der Rotation bis zu einem dritten, expandierten Zustand aufrichtbar ist.
4. Rotor nach einem der Aspekte 1 bis 3, dadurch gekennzeichnet, dass dieser nabenlos ausgebildet ist und die Strebe(n) sich bezüglich der Rotationsachse in Radialrichtung von einem ersten Achsabstand bis zu einem zweiten Achsabstand erstrecken.
5. Rotor nach einem der Aspekte 1 bis 3, dadurch gekennzeichnet, dass das wenigstens eine Schaufelblatt mit einer Nabe (24, 35, 35a, 35b, 35c, 35d, 35e, 35f, 35g, 81, 91) verbunden und gegenüber dieser schwenkbar ist.
6. Rotor nach Aspekt 5, dadurch gekennzeichnet, dass die wenigstens eine Strebe (52, 53) sich bis in die Nabe (35d) hinein erstreckt.
7. Rotor nach Aspekt 5, dadurch gekennzeichnet, dass die wenigstens eine Strebe (57) sich radial von einem ersten Achsabstand (57) radial außerhalb der Nabe (35e) bis zu einem zweiten Achsabstand (60) erstreckt.
8. Rotor nach Aspekt 1 oder einem der folgenden, dadurch gekennzeichnet, dass eine Mehrzahl von Streben (25, 26, 27, 30, 31, 32, 33) vorgesehen ist, die untereinander verbunden sind.
9. Rotor nach Aspekt 8, dadurch gekennzeichnet, dass die Streben (25, 26, 27, 30, 31, 32, 33) untereinander innerhalb der rotationsachsnahen Hälfte ihrer radialen Ausdehnung, insbesondere an ihren rotationsachsnahen Enden, miteinander verbunden sind.
10. Rotor nach Aspekt 9, dadurch gekennzeichnet, dass die Streben (25, 26, 27, 30, 31, 32, 33) ausschließlich innerhalb der rotationsachsnahen Hälfte ihrer radialen Ausdehnung miteinander verbunden sind.
11. Rotor nach Aspekt 1 oder einem der folgenden, dadurch gekennzeichnet, dass die Streben (25, 26, 27, 30, 31, 32, 52, 53, 57, 58, 62, 63, 67, 95) mit dem Material des Schaufelblattkörpers umspritzt sind.
12. Rotor nach Aspekt 1 oder einem der folgenden, dadurch gekennzeichnet, dass die Streben (62, 63) in Ausnehmungen (64, 65) des Schaufelblattkörpers angeordnet sind, welche in Längsund/oder in Querrichtung der jeweiligen Strebe größer sind als die Außenmaße der Strebe.
13. Rotor nach Aspekt 1 oder einem der folgenden, dadurch gekennzeichnet, dass wenigstens eine Strebe auf ihrer der Druckseite des Schaufelblattes zugewandten Fläche und/oder auf ihrer der Saugseite zugewandten Fläche an dem Material des Schaufelblattkörpers haftet.
14. Rotor nach Aspekt 1 oder einem der folgenden, dadurch gekennzeichnet, dass jeweils zwei benachbarte Streben (30, 31, 32, 33) paarweise in ihrem radial äußeren Bereich miteinander verbunden sind.
15. Rotor nach Aspekt 1 oder einem der folgenden, dadurch gekennzeichnet, dass die Strebe(n) (25, 26, 27, 30, 31, 32, 52, 53, 57, 58, 62, 63, 67, 95) wenigstens teilweise aus einem superelastischen Material, insbesondere einem superelastischen Polymer, oder einer Gedächtnislegierung, insbesondere Nitinol, besteht/bestehen.
16. Rotor nach Aspekt 1 oder einem der folgenden, dadurch gekennzeichnet, dass die Strebe(n) (30, 31, 32, 33) wenigstens teilweise in einem Winkel von wenigstens 90° zur Axialrichtung der Rotationsachse (29) verlaufen.
17. Rotor nach Aspekt 1 oder einem der folgenden, dadurch gekennzeichnet, dass das Schaufelblatt (74) auf seiner Druckseite wenigstens ein zugfestes Element (77, 78, 79, 80, 86, 87) in Form eines Bandes oder einer Folie aufweist, das an wenigstens einem radial äußeren Befestigungspunkt (83, 85) und wenigstens einem radial inneren Befestigungspunkt (83, 85) mit einer Strebe und/oder dem Schaufelblattkörper verbunden ist.
18. Rotor nach Aspekt 1 oder einem der folgenden, dadurch gekennzeichnet, dass der Rotor wenigstens auf der Druckseite des Schaufelblattes wenigstens ein zugfestes Element (79, 80) in Form eines Bandes oder einer Folie aufweist, das einerseits an einer Strebe und/oder dem Schaufelblattkörper (74) in radialem Abstand von der Rotationsachse sowie andererseits an einer Nabe (81) befestigt ist.
19. Rotor nach Aspekt 17 oder 18, dadurch gekennzeichnet, dass das zugfeste Element (77, 78, 79, 80, 86, 87) Glasfasern oder Polycarbonatfasern enthält.
20. Rotor nach Aspekt 17, 18 oder 19, dadurch gekennzeichnet, dass das zugfeste Element (86, 87) wenigstens teilweise auf der Oberfläche einer Strebe oder des Schaufelblattkörpers (74) haftet.
21. Rotor nach Aspekt 20, dadurch gekennzeichnet, dass das zugfeste Element aufgeklebt, aufgespritzt oder aufgedruckt ist.
22. Rotor nach einem der Aspekte 17 bis 21, dadurch gekennzeichnet, dass das zugfeste Element (86, 87) im ersten Zustand des Rotors mäanderförmig verläuft.
23. Rotor nach Aspekt 1 oder einem der folgenden mit Ausnahme von Aspekt 4, dadurch gekennzeichnet, dass das Schaufelblatt (36b, 36c, 36d, 337b, 37c, 37d) schwenkbar in einer mantelseitigen Ausnehmung einer hohlzylindrischen Nabe (35b, 35c, 35d) gelagert ist.
24. Rotor nach Aspekt 23, dadurch gekennzeichnet, dass das Schaufelblatt mit einem Innenende durch die Ausnehmung hindurch in den zylindrischen Hohlraum (44) der Nabe (35c) hineinragt.
25. Rotor nach Aspekt 24, dadurch gekennzeichnet, dass das Innenende im dritten Zustand mit einem Anschlag (42, 43, 55, 56) im Hohlraum der Nabe zusammenwirkt.
26. Rotor nach Aspekt 23, 24 oder 25, dadurch gekennzeichnet, dass wenigstens eine Strebe des Schaufelblattes in den zylindrischen Hohlraum der Nabe hineinragt.
27. Rotor nach Aspekt 26, dadurch gekennzeichnet, dass an dem in den Hohlraum der Nabe hineinragenden Ende der Strebe (52) ein Anschlagkörper (52a) vorgesehen ist, der mit dem Anschlag (56) im dritten Zustand zusammenwirkt.
28. Rotor nach Aspekt 1 oder einem der folgenden, dadurch gekennzeichnet, dass wenigstens eine der Streben eines Schaufelblattes als wenigstens ein mäanderförmig verlaufender strangförmiger Körper gestaltet ist.
29. Rotor nach einem der Aspekte 1 bis 27 mit Ausnahme von Aspekt 4, dadurch gekennzeichnet, dass jede der Streben (52) in einen zentralen Hohlraum der Nabe hineinragt und dort mit einem Anschlagkörper (52a) verbunden ist, der als senkrecht zur Rotationsachse liegendes Blechteil ausgebildet ist und insbesondere mit der jeweiligen Strebe einstückig verbunden ist.
30. Rotor nach einem der vorhergehenden Aspekte, insbesondere Aspekt 3, dadurch gekennzeichnet, dass das mindestens eine Schaufelblatt eine Strömungsdruckseite und eine Strömungssaugseite aufweist und mindestens eine Versteifungsstrebe so ausgeführt ist, dass bei Erreichen des dritten, expandierten Zustands die Versteifungsstrebe einen mechanisches Anschlag aufweist.
31. Rotor nach Aspekt 30, dadurch gekennzeichnet, dass die Versteifungsstrebe auf der Strömungssaugseite geschwächt ist.
32. Rotor nach Aspekt 31, dadurch gekennzeichnet, dass die Schwächung als quer zur Versteifungsstreben-Längsrichtung angebrachte Schlitze ausgeführt ist.
33. Rotor nach einem der vorhergehenden Aspekte, dadurch gekennzeichnet, dass die Versteifungsstrebe im radial der Nabe nahen Bereich eine Versteifungskrümmung aufweist.
34. Rotor nach einem der Aspekte 31 bis 33, dadurch gekennzeichnet, dass die Versteifungsstrebe derart im Schaufelblattkörper eingebettet ist, dass die Schwächung und/oder die Versteifungskrümmung nicht äußerlich am Schaufelblattkörper sichtbar sind.
35. Rotor nach einem der vorhergehenden Aspekte, dadurch gekennzeichnet, dass die Versteifungsstreben im Bereich der Rotorvorderkante, im Bereich der Rotorhinterkante und/oder an einer anderen Stelle des Rotors angebracht sind.
36. Rotor nach einem der vorhergehenden Aspekte, dadurch gekennzeichnet, dass die Versteifungsstreben aus einem vorzugsweise ringförmigem Nabenteil sowie einem radialen Ausleger (der eigentlichen Versteifungsstrebe, die im Schaufelblattkörper ihre versteifende Wirkung zeigt) besteht.
37. Rotor nach einem der vorhergehenden Aspekte, dadurch gekennzeichnet, dass die Versteifungsstrebe aus Metall und/oder Kunststoff besteht.

## Patentansprüche

1. Radial komprimierbarer und expandierbarer Rotor (13, 13a, 13b) für eine Pumpe mit wenigstens einem Schaufelblatt (19, 20, 22, 36, 36a, 36b, 36c, 36d, 36e, 36f, 36g, 37, 37a, 37b, 37c, 37d, 37e, 37f, 74, 88, 89, 90), wobei das Schaufelblatt einen Schaufelblattkörper aufweist, dessen Material elastisch verformbar ist, sowie wenigstens eine Versteifungsstrebe (25, 26, 27, 30, 31, 32, 52, 53, 57, 58, 62, 63, 67, 95), die in das Material des Schaufelblattkörpers wenigstens teilweise eingebettet ist.

2. Rotor nach Anspruch 1, **dadurch gekennzeichnet, dass** dieser ohne äußere Krafteinwirkung einen ersten Zustand einnimmt, von dem ausgehend er durch radiale Kompression in einen zweiten Zustand überführbar ist.

3. Rotor nach Anspruch 1 oder 2, **dadurch gekennzeichnet, dass** dieser ohne äußere Krafteinwirkung einen ersten Zustand einnimmt, von dem ausgehend das wenigstens eine Schaufelblatt im Betrieb insbesondere durch den auftretenden Fluidgegendruck und/oder Zentrifugalkräfte bei der Rotation bis zu einem dritten, expandierten Zustand aufrichtbar ist.

4. Rotor nach einem der Ansprüche 1 bis 3, **dadurch gekennzeichnet, dass** dieser nabenlos ausgebildet ist und die Strebe(n) sich bezüglich der Rotationsachse in Radialrichtung von einem ersten Achsabstand bis zu einem zweiten Achsabstand erstrecken oder dass das wenigstens eine Schaufelblatt mit einer Nabe (24, 35, 35a, 35b, 35c, 35d, 35e, 35f, 35g, 81, 91) verbunden und gegenüber dieser schwenkbar ist.

5. Rotor nach Anspruch 4, **dadurch gekennzeichnet, dass** die wenigstens eine Strebe (52, 53) sich bis in die Nabe (35d) hinein erstreckt und/oder dass die wenigstens eine Strebe (57) sich radial von einem ersten Achsabstand (57) radial außerhalb der Nabe (35e) bis zu einem zweiten Achsabstand (60) erstreckt.

6. Rotor nach Anspruch 1 oder einem der folgenden, **dadurch gekennzeichnet, dass** eine Mehrzahl von Streben (25, 26, 27, 30, 31, 32, 33) vorgesehen ist, die untereinander verbunden sind.

7. Rotor nach Anspruch 6, **dadurch gekennzeichnet, dass** die Streben (25, 26, 27, 30, 31, 32, 33) untereinander innerhalb der rotationsachsnahen Hälfte ihrer radialen Ausdehnung, insbesondere an ihren rotationsachsnahen Enden, miteinander verbunden sind.

8. Rotor nach Anspruch 7, **dadurch gekennzeichnet, dass** die Streben (25, 26, 27, 30, 31, 32, 33) ausschließlich innerhalb der rotationsachsnahen Hälfte ihrer radialen Ausdehnung miteinander verbunden sind.

9. Rotor nach Anspruch 1 oder einem der folgenden, **dadurch gekennzeichnet, dass** die Streben (25, 26, 27, 30, 31, 32, 52, 53, 57, 58, 62, 63, 67, 95) mit dem Material des Schaufelblattkörpers umspritzt sind und/oder
dass die Streben(62, 63) in Ausnehmungen (64, 65) des Schaufelblattkörpers angeordnet sind, welche in Längs- und/oder in Querrichtung der jeweiligen Strebe größer sind als die Außenmaße der Strebe und/oder
dass wenigstens eine Strebe auf ihrer der Druckseite des schaufelblattes zugewandten Fläche und/oder auf ihrer der Saugseite zugewandten Fläche an dem Material des Schaufelblattkörpers haftet und/oder
dass jeweils zwei benachbarte Streben (30, 31, 32, 33) paarweise in ihrem radial äußeren Bereich miteinander verbunden sind und/oder dass die Strebe(n) (25, 26, 27, 30, 31, 32, 52, 53, 57, 58, 62, 63, 67, 95) wenigstens teilweise aus einem superelastischen Material, insbesondere einem superelastischen Polymer, oder einer Gedächtnislegierung, insbesondere Nitinol, besteht/bestehen und/oder
dass die Strebe(n) (30, 31, 32, 33) wenigstens teilweise in einem Winkel von wenigstens 90° zur Axialrichtung der Rotationsachse (29) verlaufen und/oder
dass das Schaufelblatt (74) auf seiner Druckseite wenigstens ein zugfestes Element (77, 78, 79, 80, 86, 87) in Form eines Bandes oder einer Folie aufweist, das an wenigstens einem radial äußeren Befestigungspunkt (83, 85) und wenigstens einem radial inneren Befestigungspunkt (83, 85) mit einer Strebe und/oder dem Schaufelblattkörper verbunden ist und/oder
dass der Rotor wenigstens auf der Druckseite des Schaufelblattes wenigstens ein zugfestes Element (79, 80) in Form eines Bandes oder einer Folie aufweist, das einerseits an einer Strebe und/oder dem Schaufelblattkörper (74) in radialem Abstand von der Rotationsachse sowie andererseits an einer Nabe (81) befestigt ist.

10. Rotor nach Anspruch 9, **dadurch gekennzeichnet, dass** das zugfeste Element (77, 78, 79, 80, 86, 87) Glasfasern oder Polycarbonatfasern enthält.

11. Rotor nach Anspruch 9, **dadurch gekennzeichnet, dass** das zugfeste Element (86, 87) wenigstens teilweise auf der Oberfläche einer Strebe oder des Schaufelblattkörpers (74) haftet.

12. Rotor nach Anspruch 11, **dadurch gekennzeichnet, dass** das zugfeste Element aufgeklebt, aufgespritzt oder aufgedruckt ist.

13. Rotor nach einem der Ansprüche 9 bis 12, **dadurch gekennzeichnet, dass** das zugfeste Element (86, 87) im ersten Zustand des Rotors mäanderförmig verläuft.

14. Rotor nach Anspruch 1 oder einem der folgenden mit Ausnahme von Anspruch 4, 1. Alternative, erste Alternative, **dadurch gekennzeichnet, dass** das Schaufelblatt (36b, 36c, 36d, 337b, 37c, 37d) schwenkbar in einer mantelseitigen Ausnehmung einer hohlzylindrischen Nabe (35b, 35c, 35d) gelagert ist.

15. Rotor nach Anspruch 23, **dadurch gekennzeichnet, dass** das Schaufelblatt mit einem Innenende durch die Ausnehmung hindurch in den zylindrischen Hohlraum (44) der Nabe (35c) hineinragt.
